# EUROPÄISCHE PATENTSCHRIFT

(11) **EP 0 806 182 B1**
(45) Veröffentlichungstag und Bekanntmachung des Hinweises auf die Patenterteilung: **18.09.2002**
(21) Anmeldenummer: 97107038.8
(22) Anmeldetag: 29.04.1997
(51) Int. Cl.: A61B 17/22, G10K 9/10

(54) **Handgerät zur Verwendung bei der Lithotripsie**
Hand tool for use in lithotripsy
Outil à main utilisé pour la lithotripsie

(30) Priorität: 10.05.1996 DE 19618972
(43) Veröffentlichungstag der Anmeldung: 12.11.1997
(73) Patentinhaber: Ferton Holding S.A., 2800 Delemont (CH)
(72) Erfinder: Henry, Nicole, 1261 Burtigny (CH); Hassan, Roberto, 20145 Milano (IT)
(74) Vertreter: Gauger, Hans-Peter, Dipl.-Ing.

(56) Entgegenhaltungen:
- EP-A- 0 317 507
- GB-A- 2 161 079
- GB-A- 2 277 450
- US-A- 2 542 695
- US-A- 5 176 688
- US-A- 5 449 363

## Beschreibung

Die Erfindung bezieht sich auf ein Handgerät zur Verwendung bei der Lithotripsie, insbesondere in Verbindung mit einem Lithotripter gemäß der durch die EP 0 317 507 B1 bekannten Ausbildung, mit dem eine intrakorporal durchgeführte Zertrümmerung von Körpersteinen, wie Nieren-, Harnleiter - oder Blasensteinen, unter Verwendung eines Endoskops praktiziert wird.

Bei dem aus der EP 0 317 507 B1 bekannten Lithotripter wird ein Handgerät verwendet, welches für die Halterung einer Sonde ausgebildet ist. Die Sonde überträgt als ein Wellenleiter eine Stoßwellen erzeugende Stoßenergie, die von einem in einem Führungsrohr pneumatisch angetriebenen Schlagteil durch Ausübung einer Stoßkraft gegen das proximale Ende der Sonde erhalten wird.

Die Sonde ist zur Einführung in ein Endoskop bemessen. In Abhängigkeit von der beabsichtigten Steinzertrümmerung stehen auswechselbare Sonden mit verschiedenen Größen resp. Durchmessern zur Verfügung. Für die Halterung der Sonden an dem Handgerät ist eine Schraubkappe vorgesehen, die einen Sitz für das proximale Ende einer Sonde aufweist. Das proximale Ende jeder Sonde ist mit einem vergrößerten Kopf versehen, der als Treffläche für das Schlagteil dient. Die Schraubkappe ist an einem Außengewinde mit einem Innengewinde eines zylindrischen Gehäuseteils des Handgerätes verschraubt und durch einen an der Schraubkappe angeordneten O-Ring abgedichtet. Die Hauptlänge der Sonde steht an einem Durchsteckloch der Schraubkappe nach vorne vor, um für die Steinzetrümmerung in ein Endoskop eingeführt zu werden.

Die Schraubkappe erfüllt den Zweck, ein einfaches Auswechseln der Sonden zu ermöglichen. Außerdem ist es damit möglich, die Sonden getrennt von dem Handgerät zu sterilisieren. Eine Sterilisation auch des Handgerätes setzt allerdings voraus, daß zuvor der bei abgenommener Schraubkappe offene Raum des das Schlagteil aufnehmenden Gehäuseteils durch eine separate Verschlußkappe verschlossen wird. Dieses Verschließen erfüllt dabei gleichzeitig den Zweck, daß für die Sterilisation des Handgeräts das Schlagteil nicht verloren geht. Auch wird durch eine solche Verschlußkappe eine Verschmutzung des Schlagteils und seiner Führungsbahn in dem Führungsrohr sowie damit der gesamten Antriebsseite des Schlagteils vermieden.

Der Erfindung liegt die Aufgabe zugrunde, ein zur Verwendung bei der Lithotripsie vorgesehenes Handgerät mit den vorstehenden Merkmalen derart auszubilden, daß primär bei einem Sondenwechsel eine Verschmutzungsgefahr des das Schlagteil aufnehmenden Raumteils vermieden wird und sekundär eine getrennte Sterilisation der Sonden und des Handgeräts möglich ist. Für die Sterilisation des Handgerätes soll dabei keine besondere Vorkehrung für einen vorhergehenden Verschluß dieses Raumteils getroffen werden und sichergestellt sein, daß der Antrieb des Schlagteils danach wieder unbeeinträchtigt aufgenommen werden kann.

Diese Aufgabe wird gemäß der vorliegenden Erfindung bei einem Handgerät der durch den Patentanspruch 1 angegebenen Gattung dadurch gelöst, daß zusätzlich ein Massekörper vorgesehen wird, der in das distale Ende des das Schlagteil führenden Führungsrohres abgedichtet eingesetzt ist und die Übertragung der Stoßkraft von dem Schlagteil auf die Sonde vermittelt.

Durch die Anordnung eines solchen stoßübertragenden Massekörpers wird bei einer passenden Auswahl der Masse und des Materials dieses Massekörpers im Verhältnis zu dem Schlagteil keine Benachteiligung bei der Erzeugung der Stoßwellen erhalten, die aus der Übertragung der Stoßenergie an die Sonde resultieren. Der Massekörper folgt nämlich insoweit den Newton'schen Axiomen, gemäß welchen u.a. jeder Körper in einem Zustand der Ruhe oder der gleichförmigen, geradlinigen Bewegung verharrt, solange keine Kräfte auf ihn einwirken. Der gemäß der Erfindung in das Führungsrohr für das Schlagteil eingesetzte Massekörper ergibt daher für die maßgebliche Führungslänge des Schlagteils einen nahezu idealen Dichtungskörper, weil er beim Auftreffen des Schlagteils in einer Ruhestellung verharrt und die auf ihn einwirkende Stoßkraft nach dem Reaktionsprinzip so an die Sonde weiterleitet, als ob die Stoßkraft von dem Schlagteil direkt auf die Sonde einwirken würde.

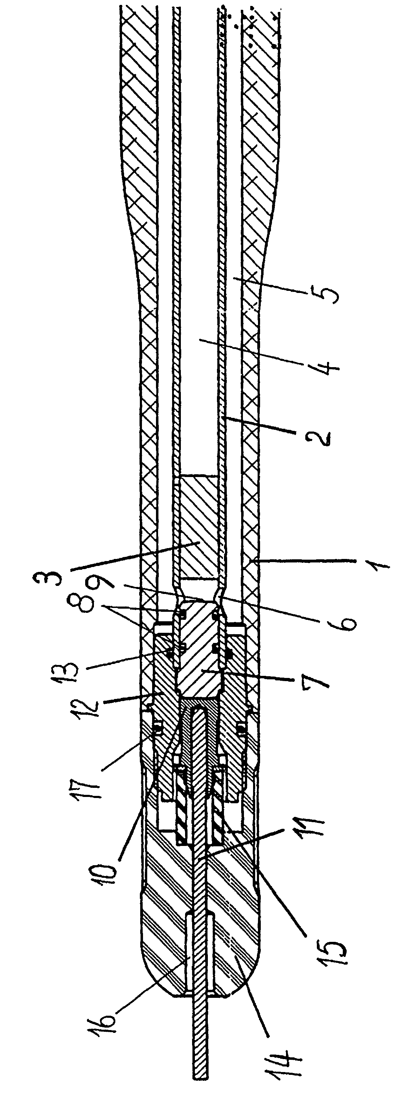

Weitere Merkmale des erfindungsgemäßen Handgerätes sind in den einzelnen Ansprüchen angegeben und werden aus der folgenden Beschreibung in Verbindung mit der Zeichnung erkennbar, die eine Schnittdarstellung nur des Befestigungsendes für eine Sonde bei einem Handgerät zeigt, das zur Verwendung bei der Lithotripsie vorgesehen ist.

Das zur Verwendung bei der Lithotripsie vorgesehene Handgerät gemäß der vorliegenden Erfindung erfüllt grundsätzlich dieselbe Funktion wie das Handgerät bei dem Lithotripter gemäß der EP 0 317 507 B1. Wie bei dem bekannten Handgerät ist daher auch hier ein zylindrischer Gehäuseteil 1 des Handgerätes für eine koaxiale Aufnahme eines Führungsrohres 2 vorgesehen.

In dem Führungsrohr 2 ist ein Schlagteil 3 angeordnet, das durch einen pneumatischen Antrieb hin- und herbewegt wird. Der pneumatische Antrieb wirkt reversierend zwischen dem Innenraum 4 des Führungsrohres 2 und dem umgebenden ringförmigen Außenraum 5, mit welchem an dem distalen Ende des Führungsrohres 2 eine Verbindung über den Luftdurchtritt steuernde Öffnungen 6 besteht. Für weitere Einzelheiten des pneumatischen Antriebs für das Schlagteil 3 kann auf die EP 0 317 507 B1 verwiesen werden. Anstelle eines pneumatischen Antriebs kann für das Schlagteil auch ein hydraulischer oder ein elektromagnetischer Antrieb realisiert sein.

In das distale Ende des Führungsrohres 2 ist ein Massekörper 7 mit einer Abdichtung durch zwei O-Ringe 8 satt eingesetzt. Dieser Massekörper 7 hat eine mit dem Schlagteil 3 etwa übereinstimmende Masse und besteht aus dem gleichen Material wie das Schlagteil. Wenn daher das Schlagteil bei seinem Vorwärtsantrieb an einer Treffläche 9 gegen diesen Massekörper 7 mit einer Stoßkraft einwirkt, dann wird diese Stoßkraft an das mit einem vergrößerten Kopf 10 ausgebildete proximale Ende einer Sonde 11 übertragen. Aus dieser Übertragung resultiert eine Stoßenergie, die in der Sonde 11 Stoßwellen erzeugt, welche über das distale Ende der Sonde für eine Steinzertrümmerung genutzt werden. Die Übertragung der von dem Schlagteil 3 ausgeübten Stoßkraft final an die Sonde 11 findet dabei etwa in der gleichen Größenordnung statt wie dann, wenn das Schlagteil 3 direkt auf das proximale Ende der Sonde 11 auftreffen würde. Der Massekörper 7 verharrt nämlich bei dem Auftreffen des Schlagteils 3 in einer Ruhestellung, sodaß sich in Übereinstimmung mit dem Newton'schen Reaktionsprinzip eine direkte Weiterleitung der Stoßkraft an die Sonde 11 ergibt. Durch die während der Stoßausübung beibehaltene Ruhestellung des Massekörpers 7 ergibt der Massekörper daneben gleichzeitig eine optimale Abdichtung für das distale Ende des Führungsrohres 2.

Diese Dichtungswirkung des Massekörpers 7 wird weiterhin mit einer Führungsbüchse 12 ergänzt, die an einem Außengewinde mit einem Innengewinde des Gehäuseteils 1 verschraubt ist. Die Führungsbüchse 12 ist im Bereich dieser Verschraubung auf das distale Ende des Führungsrohres 2 aufgeschoben und gegen dieses Ende durch einen weiteren O-Ring 13 abgedichtet. Die Führungsbüchse 12 umgibt auch das über das Führungsrohr 2 vorstehende Ende des Massekörpers 7 sowie das proximale Ende der Sonde 11 resp. deren Kopfteil 10, über welchen die Sonde direkt an dem Massekörper 7 anliegt.

Mit der mit dem Gehäuseteil 1 verschraubten Führungsbüchse 12 ist über zusammenwirkende Außen- und Innengewinde eine Schraubkappe 14 verschraubt. Die Schraubkappe 14 weist einen Sitz für eine an dem proximalen Ende der Sonde 11 angeordnete Silikondichtung 15 auf und ist mit einem Durchsteckloch 16 versehen, durch welches hindurch die proximale Teillänge der Sonde 11 verläuft. Die zur Einführung in ein Endoskop bezweckte Hauptlänge der Sonde steht andererseits über die Schraubkappe nach vorne vor. Auch die Schraubkappe 14 ist über einen O-Ring 17 an der Führungsbuchse 12 abgedichtet, und eine entsprechende Abdichtung (nicht gezeigt) der Führungsbuchse 12 ist auch in Bezug auf den Gehäuseteil 1 vorgesehen.

Nach dem Abschrauben der Schraubkappe 14 von der Führungsbuchse 12 kann die Sonde 11 an ihrem Kopfteil 10 aus der Führungsbuchse 12 herausgezogen werden. Die Sonde 11 kann dann getrennt von dem Handgerät sterilisiert werden. Für eine Sterilisation des Handgerätes wird andererseits das distale Ende des Führungsrohres 2 durch den Massekörper 7 abgedichtet sowie im Zusammenwirken mit der Führungsbuchse 12 auch das distale Ende des Gehäuseteils 1, sodaß die gesamte Antriebseinheit für das Schlagteil 3 gegen eine Verschmutzungsgefahr verschlossen ist. Durch den Massekörper 7 wird gleichzeitig verhindert, daß das Schlagteil 3 bei entfernter Schraubkappe 14 verloren geht.

Über eine Auswahl einer zu dem Schlagteil unterschiedlichen Masse und/oder eines unterschiedlichen Materials für den Massekörper kann die von dem Schlagteil auf die Sonde vermittelte Stoßkraft in Übereinstimmung mit vorbestimmten anwendungstechnischen und medizinischen Vorstellungen individuell gestaltet werden. Auch erlaubt es der Massekörper, den Übertragungsweg der Stoßkraft von dem Schlagteil auf die Sonde anders zu gestalten als er hier am Beispiel des Handgerätes bei dem Lithotripter gemäß der EP 0 317 507 B1 beschrieben wurde.

Das Funktionsprinzip des Massekörpers, gleichzeitig als ein Dichtungsorgan und als ein Übertragungsorgan für Stoßkräfte zu wirken, kann außer bei der Lithotripsie auch bspw. in der Orthopädie dort anwendbar sein, wo solche Stoßkräfte zur Übertragung kommen. Bei der Übertragung der Stoßkräfte dann unter zusätzlicher Einschaltung eines solchen Massekörpers ist es von besonderer Bedeutung, daß der Massekörper in einer Ruhestellung verharrt und so durch ihn das proximale Ende der Sonde von einem Verschleiß verschont wird.

Schließlich können die Vorteile des Massekörpers auch für beliebig andere Antriebsarten des Schlagteils realisiert werden. Die Formgebungen des Massekörpers und des Schlagteils sind dabei nur von sekundärer Bedeutung, solange nur eine ungestörte Übertragung der Stoßenergie an die Sonde erreichbar ist.

## Patentansprüche

1. Handgerät zur Verwendung bei der Lithotripsie, mit
- einer Sonde (11), die als ein Wellenleiter zur Übertragung einer Stoßwellen erzeugenden Stoßenergie vorgesehen und zur Einführung in ein Endoskop bemessen ist;
- einem Schlagteil (3), das in einem Führungsrohr (2) des Gerätes hin- und herbeweglich angetrieben wird und die durch die Sonde (11) übertragene Stoßenergie durch die Ausübung einer Stoßkraft gegen das proximale Ende der Sonde erzeugt;
- einer gegen ein Gehäuseteil (1) und gegen das Führungsrohr (2) abgedichteten Halterung (12, 14) für eine auswechselbare Anordnung der Sonde (11) und eine Festlegung des proximalen Endes der Sonde an dem Handgerät;
**gekennzeichnet durch**
- einen Massekörper (7), der in das distale Ende des das Schlagteil (3) führenden Führungsrohres (2) abgedichtet eingesetzt ist und die Übertragung der Stoßkraft von dem Schlagteil (3) auf die Sonde (11) vermittelt.

2. Handgerät nach Anspruch 1, bei welchem das proximale Ende (10) der Sonde (11) mit dem Massekörper (7) direkt in Berührung gehalten ist.

3. Handgerät nach Anspruch 1 oder 2, bei welchem der Massekörper (7) eine etwa gleiche Masse wie das Schlagteil (3) hat.

4. Handgerät nach Anspruch 1 oder 2, bei welchem der Massekörper (7) eine zu dem Schlagteil (3) unterschiedliche Masse hat.

5. Handgerät nach Anspruch 4, bei welchem die Masse des Massekörpers (7) größer ist als die Masse des Schlagteils (3).

6. Handgerät nach Anspruch 4, bei welchem die Masse des Massekörpers (7) kleiner ist als die Masse des Schlagteils (3).

7. Handgerät nach einem der Ansprüche 1 bis 6, bei welchem der Massekörper (7) aus demselben Material wie das Schlagteil (3) besteht.

8. Handgerät nach einem der Ansprüche 1 bis 7, bei welchem der Massekörper (7) etwa zylindrisch ausgebildet und durch wenigstens einen O-Ring (8) an dem Führungsrohr (2) abgedichtet ist.

9. Handgerät nach einem der Ansprüche 1 bis 8, bei welchem das proximale Ende (10) der Sonde (11) in eine durch die Halterung an dem Gehäuseteil (1) des Gerätes festgelegte Führungsbüchse (12) eingesetzt ist, welche auf das distale Ende des Führungsrohres (2) abgedichtet (13) aufgeschoben ist.

10. Handgerät nach Anspruch 9, bei welchem die Führungsbüchse (12) an einem Außengewinde mit einem Innengewinde des Gehäuseteils (1) verschraubt ist.

11. Handgerät nach einem der Ansprüche 1 bis 10, bei welchem die Halterung mit einer einen Sitz für das proximale Ende (10) der Sonde (11) aufweisenden und mit einem Durchsteckloch (16) für die Sonde (11) versehenen Schraubkappe (14) ausgebildet ist, wobei die Schraubkappe (14) an einem Innengewinde mit einem Außengewinde der Führungsbüchse (12) verschraubt ist.

## Claims

1. Handpiece for use in lithotripsy, comprising
- a probe (11) which is provided as a wave guide for transmission of an impact energy producing shock waves and which is dimensioned such as to be inserted into an endoscope;
- an impact member (3) which is driven in a reciprocating manner inside of a guide tube (2) of the device and which produces the impact energy as transmitted through the probe (11) by effecting an impact force against a proximal end of the probe;
- a holding means (12, 14) which is sealed against a portion (1) of a casing and against the guide tube (2) and which is adapted for exchangeably holding the probe (11) and for fixing the proximal end of the probe on the handpiece;
**characterised by**
- a mass body (7) which is inserted in a sealed manner into the distal end of the guide tube (2) for guiding the impact member (3) and which is arranged for transmitting the impact force from the impact member (3) to the probe (11).

2. Handpiece according to claim 1, wherein the proximal end (10) of the probe (11) is in direct contact with the mass body (7).

3. Handpiece according to claim 1 or 2, wherein the mass body (7) has approximately the same mass as the impact member (3).

4. Handpiece according to claim 1 or 2, wherein the mass body (7) has a mass different from the impact member (3).

5. Handpiece according to claim 4, wherein the mass of the mass body (7) is greater than the mass of the impact member (3).

6. Handpiece according to claim 4, wherein the mass of the body (7) is smaller than the mass of the impact member (3).

7. Handpiece according to any of the claims 1 to 6, wherein the mass body (7) is of the same material as the impact member (3).

8. Handpiece according to any of the claims 1 to 7, wherein the mass body (7) is substantially cylindrical and is sealed against the guide tube (2) by at least one O-ring (8).

9. Handpiece according to any of the claims 1 to 8, wherein the proximal end (10) of the probe (11) is inserted into a guide bush (12) which is fixed through the holding means on the portion (1) of the casing of the device, said guide bush being sealingly (13) pushed on-to the distal end of the guide tube (2).

10. Handpiece according to claim 9, wherein the guide bush (12) is screwed with a male thread to a female thread of the portion (1) of the casing.

11. Handpiece according to any of the claims 1 to 10, wherein the holding means comprises a screw cap (14) forming a seat for the proximal end (10) of the probe (11) and being provided with a throughhole (16) for the probe (11) whereby the screw cap (14) is screwed with a female thread to a male thread of the guide bush (12).

## Revendications

1. Manipulateur manuel pour l'emploi dans la lithotripsie, comprenant
- une sonde (11) disposé en tant que guide d'ondes pour la transmission d'une énergie de choc engendrant des ondes de choc et qui est dimensionné pour l'introduction dans un endoscope;
- un élément de chasse (3), qui est entraîné pour un mouvement va-et-vient dans un tube de guidage (2) et qui engendre l'énergie de choc transmise par ladite sonde (11) par l'exercice d'une force de choc sur l'extrémité proximale de ladite sonde;
- une attache (12, 14) rendue étanche relativement à un élément de boîtier (1) et ledit tube de guidage (2) pour l'arrangement échangeable de ladite sonde (11) et pour la fixation de l'extrémité proximale de ladite sonde au manipulateur;
**caractérisé par**
- un corps de masse (7) introduit, à étanchéité, dans l'extrémité distale dudit tube de guidage (2), qui guide ledit élément de chasse (3) et qui communique la transmission de la force de choc dudit élément de chasse (3) sur ladite sonde (11).

2. Manipulateur selon la revendication 1, dans lequel ladite extrémité proximale (10) de ladite sonde (11) est tenue en contact direct avec ledit corps de masse (7).

3. Manipulateur selon la revendication 1 ou 2, dans lequel ledit corps de masse (7) a la même masse environ que ledit élément de chasse (3).

4. Manipulateur selon la revendication 1 ou 2, dans lequel ledit corps de masse (7) a une masse différente de la masse dudit élément de chasse (3).

5. Manipulateur selon la revendication 4, dans lequel la masse dudit corps de masse (7) est plus grande que la masse dudit élément de chasse (3).

6. Manipulateur selon la revendication 4, dans lequel la masse dudit corps de masse (7) est plus petite que la masse dudit élément de chasse (3).

7. Manipulateur selon une quelconque des revendications 1 à 6, dans lequel ledit corps de masse (7) est fait du même matériau que ledit élément de chasse (3).

8. Manipulateur selon une quelconque des revendications 1 à 7, dans lequel ledit corps de masse (7) a une configuration cylindrique environ et est rendu étanche audit tube de guidage (2) moyennant au moins un anneau torique d'étanchéité.

9. Manipulateur selon une quelconque des revendications 1 à 8, dans lequel ladite extrémité proximale (10) de ladite sonde (11) est insérée dans une douille de guidage (12) fixée audit élément de boîtier (1) du dispositif moyennant ladite attache, cette douille étant poussée, à étanchéité, sur l'extrémité distale dudit tube de guidage (2).

10. Manipulateur selon la revendication 9, dans lequel ladite douille de guidage (12) est vissé par un filet extérieur dans un filet femelle dudit élément de boîtier (1).

11. Manipulateur selon une quelconque des revendications 1 à 10, dans lequel ladite attache est conçue de façon qu'elle présente un bouchon fileté ayant une siège pour l'extrémité proximale (10) de ladite sonde (11) et muni d'un trou traversant (16) pour ladite sonde (11), ce bouchon fileté (14) étant vissé par un filet femelle dans un filet extérieur de ladite douille de guidage (12).
